# FASCICULE DE BREVET EUROPEEN

(11) **EP 3 938 357 B1**
(45) Date de publication et mention de la délivrance du brevet: **15.05.2024**
(21) Numéro de dépôt: 20725866.6
(22) Date de dépôt: 10.03.2020
(51) Int. Cl.: C07D 265/16, C07D 407/06, C08G 73/02

(54) **PROCEDE DE FABRICATION D'UN MONOMERE POLYBENZOXAZINE**
VERFAHREN ZUR HERSTELLUNG EINES POLYBENZOXAZIN-MONOMERS
PROCESS FOR PRODUCING A POLYBENZOXAZINE MONOMER

(30) Priorité: 15.03.2019 FR 1902698
(43) Date de publication de la demande: 19.01.2022
(73) Titulaire: ArianeGroup SAS, 78130 Les Mureaux (FR); Centre national de la recherche scientifique, 75016 Paris (FR)
(72) Inventeur: TAVERNIER, Romain, 80600 DOULLENS (FR); GRANADO, Lérys, 11000 CARCASSONNE (FR); DAVID, Ghislain, 34080 MONTPELLIER (FR); CAILLOL, Sylvain, 34090 MONTPELLIER (FR); FOYER, Gabriel, 33700 MERIGNAC (FR)
(74) Mandataire: Cabinet Beau de Loménie
(86) Numéro de dépôt international: PCT/FR2020/050486
(87) Numéro de publication internationale: WO 2020/188183

(56) Documents cités:
- WO-A1-01/34581
- WO-A1-92/16470
- US-A1- 2017 158 877
- SEISHI OHASHI ET AL: "Synthesis and ring-opening polymerization of 2-substituted 1,3-benzoxazine: the first observation of the polymerization of oxazine ring-substituted benzoxazines", POLYMER CHEMISTRY, vol. 7, no. 46, janvier 2016 (2016-01), pages 7177-7184, XP055626808, ISSN: 1759-9954, DOI: 10.1039/C6PY01686C
- ZILONG TANG ET AL: "Efficient Synthesis of 2,3-Disubstituted-1,3-benzoxazines by Chlorotrimethylsilane-Mediated Aza-Acetalizations of Aromatic Aldehydes : TMSCl-Catalyzed Synthesis of 2,3-Disubstituted-1,3-benzoxazines", JOURNAL OF HETEROCYCLIC CHEMISTRY, août 2013 (2013-08), pages n/a-n/a, XP055626764, US ISSN: 0022-152X, DOI: 10.1002/jhet.1590
- ZI-LONG TANG ET AL: "Synthesis and fungicidal activity of novel 2-aryl-3-(1,3,4-thiadiazolyl)-6(8)-methyl- 1,3-benzoxazines", BIOORGANIC & MEDICINAL CHEMISTRY LETTERS, vol. 25, no. 16, août 2015 (2015-08), pages 3378-3381, XP055626759, AMSTERDAM, NL ISSN: 0960-894X, DOI: 10.1016/j.bmcl.2015.05.010
- HAJIME KANATOMI ET AL: "Reaction of Salicylamine with [alpha]-Dicarbonyl Compounds. II. Formation of 2,2'-Bibenz-1,3-oxazines", BULLETIN OF THE CHEMICAL SOCIETY OF JAPAN, vol. 43, no. 1, janvier 1970 (1970-01), pages 226-231, XP055626769, JP ISSN: 0009-2673, DOI: 10.1246/bcsj.43.226

## Description

### Domaine Technique

L'invention concerne un procédé de fabrication d'un monomère polybenzoxazine et la réticulation de ce monomère afin de former un produit réticulé ayant des propriétés de charbonnement et de stabilité thermique améliorées. Ce produit réticulé peut notamment être utilisé en tant que résine ablative pour la constitution d'une tuyère de propulseur ou d'un corps de rentrée atmosphérique.

### Technique antérieure

Il est connu de réaliser des protections thermiques ablatives de tuyères de propulseurs ou de corps de rentrée atmosphérique à partir de résines phénoliques synthétisées à partir de formaldéhyde et de phénol, afin d'obtenir des densités d'aromatiques et de réticulation élevées, et donc un taux de coke important. Le formaldéhyde et le phénol sont toutefois des composés classés Cancérigène Mutagène Reprotoxique (CMR) de catégorie 1B et 2 dont il serait souhaitable de limiter l'usage, notamment pour anticiper d'éventuelles interdictions en Union Européenne. En outre, la réaction du phénol et du formaldéhyde est une polycondensation pendant laquelle de l'eau est produite. Cette eau peut se retrouver piégée au sein du produit fini, conduisant à une baisse des performances. Dans l'optique de résoudre ce problème, des développements ont été poursuivis afin d'obtenir des résines ablatives par réticulation de benzoxazines. Ces développements réduisent le piégeage de l'eau dans le produit fini mais les solutions de la littérature, fournissant un produit fini avec des propriétés de stabilité thermique et de charbonnement compatibles d'une application en tant que résine ablative, mettent en jeu des benzoxazines obtenues par réaction du phénol avec le formaldéhyde et une amine, et se basent donc sur la mise en oeuvre de composés classés CMR. De plus, lorsque ces benzoxazines sont synthétisées sans phénol, elles présentent des propriétés de stabilité thermique et de charbonnement peu performantes.

On connaît la publication SEISHI OHASHI et al. (Polymer Chemistry, vol.7, no.46, janvier 2016, pages 7177-7184, DOI : 10.1039/C6PY01686C) qui divulgue la synthèse d'une polybenzoxazine.

Il serait par conséquent souhaitable de disposer d'une nouvelle voie de synthèse de benzoxazines limitant l'emploi de composés CMR, et s'affranchissant en particulier de l'utilisation de formaldéhyde.

Il serait en outre souhaitable de disposer d'une nouvelle voie de synthèse de matériaux présentant des propriétés de stabilité thermique et de charbonnement adaptées à la réalisation de protections thermiques ablatives pour tuyères de propulseurs ou corps de rentrée atmosphérique.

### Exposé de l'invention

L'invention concerne un procédé de fabrication d'un monomère polybenzoxazine C via réaction d'une polyamine A avec un aldéhyde B; les formules A, B et C étant comme définies dans la revendication 1.

L'invention fournit une nouvelle voie de synthèse de polybenzoxazines limitant l'utilisation de composés CMR, et s'affranchissant en particulier de l'emploi de formaldéhyde. En outre, le produit obtenu par réticulation du monomère polybenzoxazine de formule C présente de hautes propriétés de stabilité thermique et de charbonnement, compatibles d'une application en tant que résine ablative pour tuyère de propulseur ou corps de rentrée atmosphérique. En outre, le monomère polybenzoxazine de formule C présente un caractère liquide à température modérée et sans avoir recours à un solvant aromatique ou halogéné. Il présente donc un emploi aisé dans des procédés comme le moulage par transfert de résine (« Resin Transfer Molding » ; « RTM »).

En particulier, A₂ peut être un groupement xylylène substitué ou non substitué.

Une telle caractéristique permet d'améliorer davantage encore la conversion en monomère polybenzoxazine de formule C.

Selon une variante, A₂ peut être choisi parmi les chaînes hydrocarbonées linéaires ou ramifiées, saturées, substituées ou non substituées, non interrompues.

Dans un exemple de réalisation, n^{a} est égal à 0.

Dans la variante où n^{a} est non nul, R₁^{a} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués.

Dans un exemple de réalisation, le procédé peut comprendre, avant la condensation, l'obtention de la polyamine de formule A, cette obtention comprenant :
- une réaction d'addition d'une polyamine de formule [NH₂]_{N1}-A₂ sur un aldéhyde de formule B2 afin de former une imine de formule A3, et
- une réaction de réduction de l'imine de formule A3 en polyamine de formule A, les formules B2 et A3 étant fournies ci-dessous :

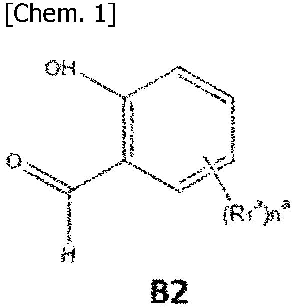

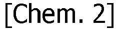

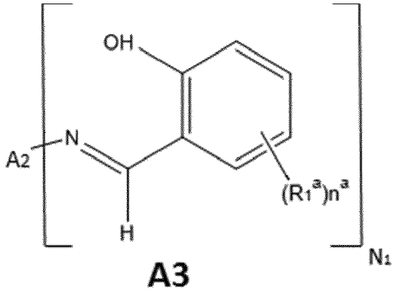

L'amine de formule [NH₂]_{N1}-A₂ est par exemple choisie parmi les composés suivants : la méta-xylylènediamine. L'aldéhyde de formule B2 peut par exemple être le salicylaldéhyde.

L'aldéhyde de formule B peut être un monoaldéhyde, c'est-à-dire ne comprendre qu'une seule fonction aldéhyde.

B₁ est choisi parmi les groupes carbocycliques aromatiques ou hétérocycliques aromatiques, monocycliques ou polycycliques, substitués ou non substitués.

Le choix d'un tel aldéhyde permet avantageusement d'obtenir après réticulation du monomère polybenzoxazine de formule C un produit présentant des propriétés de stabilité thermique et de charbonnement améliorées.

En particulier, B₁ peut être un cycle benzénique substitué ou non substitué.

D'une manière générale et quel que soit l'exemple de réalisation considéré, N₁ peut être un entier égal à 2. Dans ce cas, la polyamine de formule A est une diamine. N₁ peut en variante prendre d'autres valeurs, N₁ peut en particulier être égal à 3 ou plus.

Quel que soit le mode de réalisation considéré, la condensation peut être réalisée en portant le mélange de la polyamine de formule A avec l'aldéhyde de formule B au reflux. On peut réaliser la condensation dans le toluène, le méthanol, l'éthanol ou encore sans solvant.

L'invention concerne également un procédé de fabrication d'un produit réticulé comprenant la réticulation du monomère polybenzoxazine de formule C obtenu par mise en oeuvre du procédé décrit plus haut.

Ce produit réticulé présente un haut taux de coke et une haute stabilité thermique, qui le rendent compatible d'une application en tant que résine ablative pour la fabrication d'une tuyère de propulseur ou d'un corps de rentrée atmosphérique.

La réticulation peut être effectuée en imposant une température supérieure ou égale à 130°C, par exemple comprise entre 180°C et 250°C.

Dans un exemple de réalisation, il y a réticulation d'un mélange comprenant le monomère polybenzoxazine de formule C et un monomère monobenzoxazine additionnel de formule D, la formule D étant fournie ci-dessous : 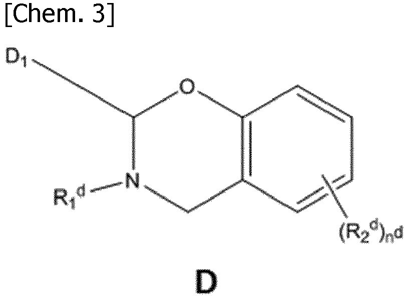 formule dans laquelle :
R₁^{d} est choisi parmi : les groupes furfuryles, substitués ou non substitués, les groupes carbocycliques ou hétérocycliques saturés, insaturés ou aromatiques, monocycliques ou polycycliques, substitués ou non substitués, les groupes aralkyles substitués ou non substitués, les chaînes hydrocarbonées linéaires ou ramifiées, saturées ou insaturées, substituées ou non substituées, interrompues ou non par un ou plusieurs hétéroatomes ou par un ou plusieurs groupes carbocycliques ou hétérocycliques saturés, insaturés ou aromatiques, monocycliques ou polycycliques, substitués ou non substitués,
R₂^{d} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués, n^{d} est un entier compris entre 0 et 2,
D₁ est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes,
et, lorsqu'ils sont substitués, chacun de R₁^{d}, R₂^{d} et D₁ étant substitué par l'un au moins des groupements suivants : hydroxyméthyle, méthyle, acide carboxylique.

Le fait d'adjoindre le monomère monobenzoxazine additionnel de formule D permet de faciliter davantage encore l'obtention d'un milieu liquide comprenant le monomère polybenzoxazine de formule C à température modérée et sans avoir recours à un solvant aromatique ou halogéné. Cela facilite davantage encore l'utilisation du mélange dans des procédés comme le moulage par transfert de résine (« Resin Transfer Molding » ; « RTM ») sans significativement affecter les propriétés thermiques et de charbonnement du produit réticulé obtenu.

On peut par exemple réticuler un mélange comprenant le monomère polybenzoxazine de formule C à raison de 20% à 80% en masse et de préférence de 20% à 50% en masse, et le monomère monobenzoxazine additionnel de formule D à raison de 20% à 80% en masse et de préférence de 50% à 80% en masse.

Dans un exemple de réalisation, D₁ est choisi parmi les groupes carbocycliques aromatiques ou hétérocycliques aromatiques, monocycliques ou polycycliques, substitués ou non substitués.

En particulier, D₁ peut être un cycle benzénique substitué ou non substitué.

D₁ peut en variante être une chaîne hydrocarbonée linéaire ou ramifiée. Le nombre d'atomes de carbone de cette chaîne hydrocarbonée peut varier dans de larges proportions, D₁ peut comprendre entre 1 et 20 atomes de carbone ou être un polymère.

R₁^{d} peut en particulier être choisi parmi : les groupes furfuryles, substitués ou non substitués, les groupes carbocycliques aromatiques ou hétérocycliques aromatiques, monocycliques ou polycycliques, substitués ou non substitués, les groupes aralkyles substitués ou non substitués. En particulier, R₁^{d} peut être un groupe furfuryle substitué ou non substitué. R₁^{d} est en variante une chaîne hydrocarbonée linéaire ou ramifiée. Le nombre d'atomes de carbone de cette chaîne hydrocarbonée peut varier dans de larges proportions. R₁^{d} peut par exemple être une chaîne hydrocarbonée linéaire ou ramifiée comprenant entre 1 et 20 atomes de carbone ou être un polymère.

Dans un exemple de réalisation, n^{d} est égal à 0.

Dans la variante où n^{d} est non nul, R₂^{d} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués.

Le monomère monobenzoxazine additionnel de formule D peut être obtenu par condensation entre une monoamine de formule D2 ci-dessous (ne comportant qu'une seule fonction amine) avec un aldéhyde de formule D₁-CHO.

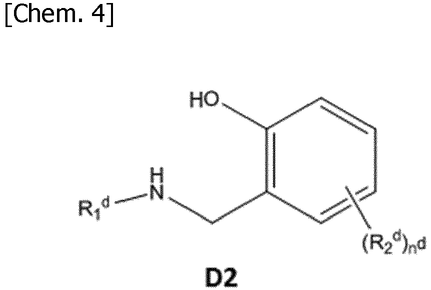

En variante, il y a réticulation d'un mélange comprenant le monomère polybenzoxazine de formule C et un monomère polybenzoxazine additionnel de formule E, la formule E étant fournie ci-dessous : 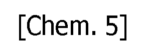 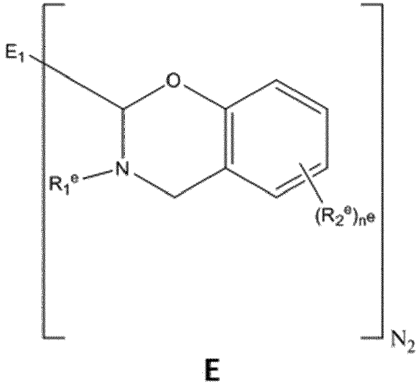
R₁^{e} est choisi parmi : les groupes furfuryles, substitués ou non substitués, les groupes carbocycliques ou hétérocycliques saturés, insaturés ou aromatiques, monocycliques ou polycycliques, substitués ou non substitués, les groupes aralkyles substitués ou non substitués, les chaînes hydrocarbonées linéaires ou ramifiées, saturées ou insaturées, substituées ou non substituées, interrompues ou non par un ou plusieurs hétéroatomes,
R₂^{e} est choisi parmi : les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{e} est un entier compris entre 0 et 2,
E₁ est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes, et N₂ est un entier supérieur ou égal à 2.

Le fait d'adjoindre le monomère polybenzoxazine additionnel de formule E permet d'augmenter davantage encore le taux de charbonnement.

On peut par exemple réticuler un mélange comprenant le monomère polybenzoxazine de formule E à raison de 20% à 80% en masse, de préférence de 20% à 50% en masse, et le monomère polybenzoxazine additionnel de formule C à raison de 20% à 80% en masse, de préférence de 50% à 80% en masse.

Quel que soit le mode de réalisation considéré, la proportion de monomère monobenzoxazine additionnel de formule D ou de monomère polybenzoxazine additionnel de formule E peut être choisie de sorte que le mélange soit liquide lorsqu'il est porté à une température comprise entre 20°C et 100°C.

A titre d'exemple, chacun de R₁^{a}, B₁, A₂, R₁^{d}, R₂^{d}, D₁, E₁, R₁^{e}, R₂^{e} peut être substitué par l'un au moins des groupements suivants : hydroxyméthyle, méthyle, acide carboxylique.

L'invention concerne également un procédé de fabrication d'une tuyère de propulseur dans lequel la tuyère est fabriquée en mettant en oeuvre un produit réticulé obtenu par le procédé décrit plus haut.

La tuyère de propulseur peut être en matériau composite. Dans ce cas, la fabrication de la tuyère peut comporter une première étape de formation d'une préforme fibreuse de la tuyère à obtenir imprégnée par le monomère polybenzoxazine de formule C obtenu comme décrit plus haut ou par un mélange comprenant ce monomère tel que décrit plus haut. Cette fabrication peut en outre comporter une deuxième étape traitement thermique de la préforme fibreuse imprégnée de manière à réticuler le monomère polybenzoxazine de formule C ou le mélange et obtenir la tuyère de propulseur.

La préforme fibreuse peut par exemple comporter des fibres de carbone, de silice, de verre ou d'un matériau céramique, par exemple de carbure de silicium. La préforme fibreuse destinée à former le renfort fibreux de la tuyère peut être formée de diverses manières (drapage de couches de tissu pré-imprégnées, par exemple). On peut ainsi en particulier draper ou bobiner des couches de tissu bi-dimensionnel ou tri-dimensionnel imprégnées sur une forme ayant une surface reproduisant la géométrie désirée d'une surface interne ou externe de la tuyère à réaliser afin d'obtenir la préforme imprégnée. En variante, on peut d'abord obtenir la préforme fibreuse de la tuyère à obtenir puis placer cette préforme dans une cavité d'injection et injecter ensuite le monomère polybenzoxazine de formule C ou le mélange comprenant ce monomère décrit plus haut dans la cavité de manière à imprégner la préforme. Dans ce cas, on peut mettre en oeuvre une technique de moulage par transfert de résine (« Resin Transfer Molding ») pour imprégner la préforme fibreuse.

L'invention concerne également un procédé de fabrication d'un corps de rentrée atmosphérique dans lequel le corps de rentrée atmosphérique est fabriqué en mettant en oeuvre un produit réticulé obtenu par le procédé décrit plus haut.

### Brève description des dessins

[Fig. 1] La figure 1 est un thermogramme DSC d'un exemple de monomère polybenzoxazine de formule C obtenu par mise en oeuvre de l'invention.
[Fig. 2] La figure 2 est un résultat d'analyse thermogravimétrique (« thermogravimetric analysis » ; « TGA ») d'un produit réticulé obtenu par réticulation de ce monomère polybenzoxazine.
[Fig. 3] La figure 3 est un thermogramme DSC d'un autre exemple de monomère polybenzoxazine de formule C obtenu par mise en oeuvre de l'invention.
[Fig. 4] La figure 4 est un résultat d'analyse thermogravimétrique d'un produit réticulé obtenu par réticulation de cet autre monomère polybenzoxazine
[Fig. 5] La figure 5 est un thermogramme DSC d'un exemple de monomère polybenzoxazine additionnel de formule E utilisable dans le cadre du procédé selon l'invention.
[Fig. 6] La figure 6 est un résultat d'analyse thermogravimétrique d'un produit réticulé obtenu par réticulation de ce monomère polybenzoxazine.

### Description des modes de réalisation

### Exemples

### Exemple 1 : synthèse d'un monomère polybenzoxazine à partir de méta-xylylène-aminométhylphénol et de benzaldéhyde puis réticulation subséquente

On fait réagir la méta-xylylènediamine avec le salicylaldéhyde dans des proportions stoechiométriques dans le méthanol au reflux pendant 2h, dans le but de former l'imine correspondante. L'imine est réduite en amine avec 2 équivalents de NaBH₄ ajouté à 0°C dans une solution de l'imine dans l'éthanol, on réalise ensuite un chauffage à reflux pendant 2 heures. Le méta-xylylène-aminométhylphénol ainsi synthétisé est dissous dans le toluène avec 2 équivalents de benzaldéhyde, puis porté au reflux dans un appareil Dean Stark, afin de retirer l'eau générée pendant la condensation. Après évaporation du solvant sous pression réduite, le produit obtenu est un liquide visqueux de couleur orangée à chaud, et solide jaune pâle à température ambiante. Le produit a été caractérisé par RMN et la structure a été confirmée.

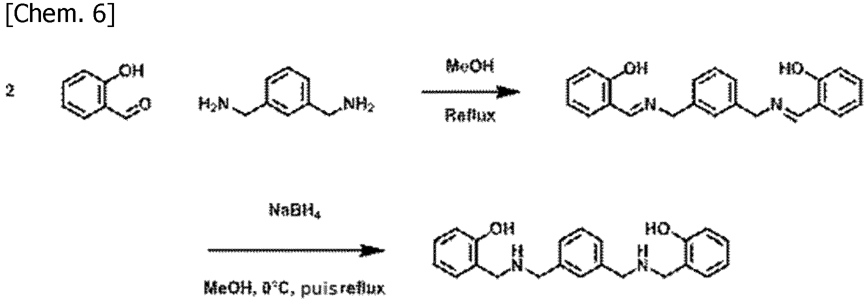

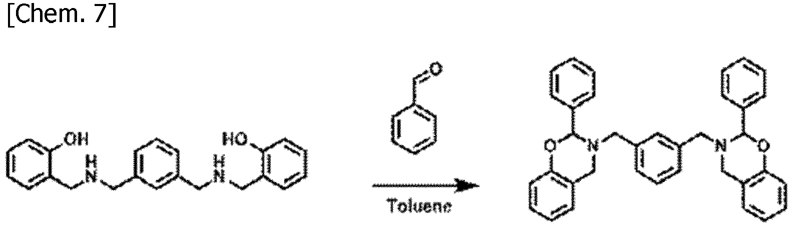

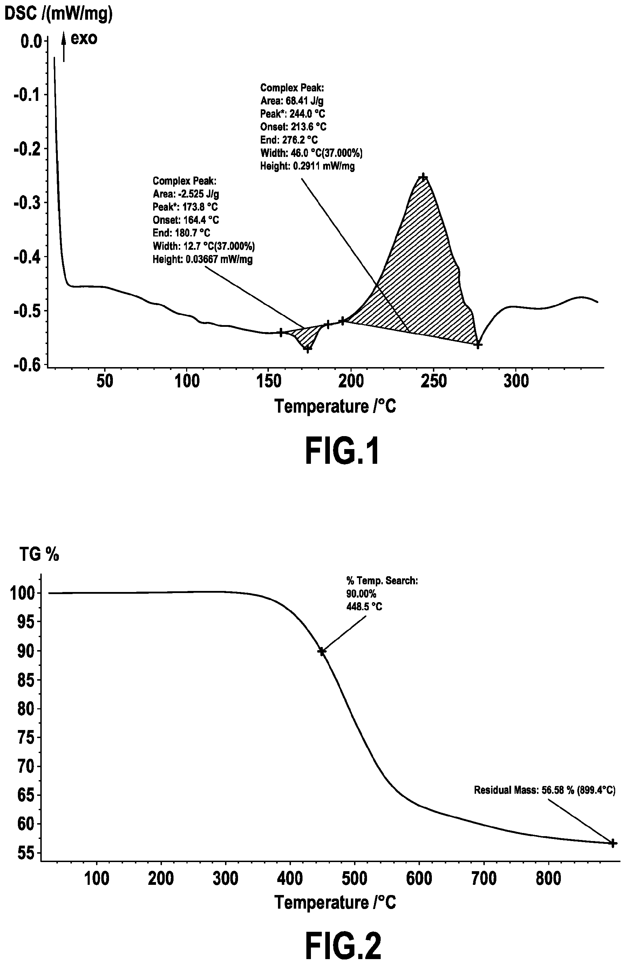

La caractérisation thermique par DSC a révélé une enthalpie de polymérisation de 68J/g. Le thermogramme DSC obtenu est fourni à la figure 1. L'analyse thermogravimétrique d'un échantillon réticulé a montré un taux de coke de 57% sous atmosphère d'azote à 900°C ainsi qu'une température de dégradation de 10% de la masse totale de 448°C. Le graphe d'analyse thermogravimétrique obtenu est fourni à la figure 2 et montre de bonnes propriétés de stabilité thermique et de charbonnement.

### Exemple 2 : synthèse d'un monomère polybenzoxazine à partir de 1,10-décanediaminomethylphénol et de benzaldéhyde puis réticulation subséquente

On fait réagir la 1,10-diaminodécane avec le salicylaldéhyde dans des proportions stoechiométriques dans le méthanol au reflux pendant 2h, dans le but de former l'imine correspondante. L'imine est réduite en amine avec 2 équivalents de NaBH₄ ajouté à 0°C dans une solution de l'imine dans l'éthanol, on réalise ensuite un chauffage à reflux pendant 2 heures. Le 1,10-décanediaminomethylphénol ainsi synthétisé est dissous dans le toluène avec 2 équivalents de benzaldéhyde, puis porté au reflux dans un appareil Dean Stark, afin de retirer l'eau générée pendant la condensation. Après évaporation du solvant sous pression réduite, le produit obtenu est un liquide visqueux de couleur orangée à chaud, et solide jaune pâle à température ambiante. Le produit a été caractérisé par RMN et la structure a été confirmée.

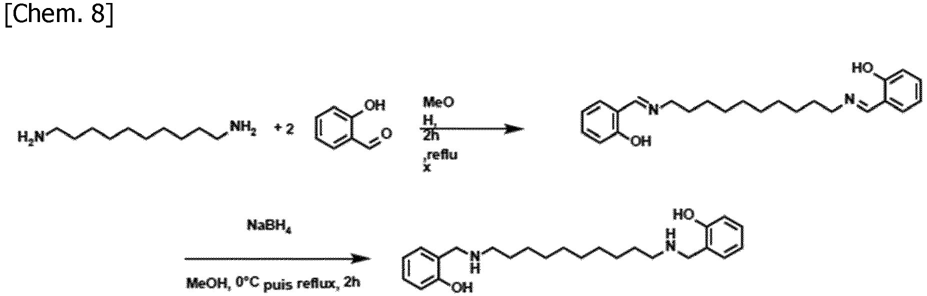

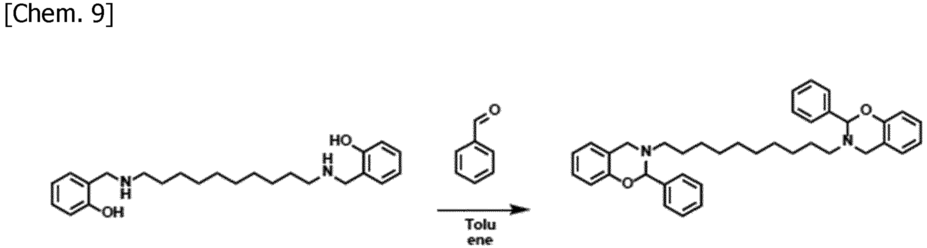

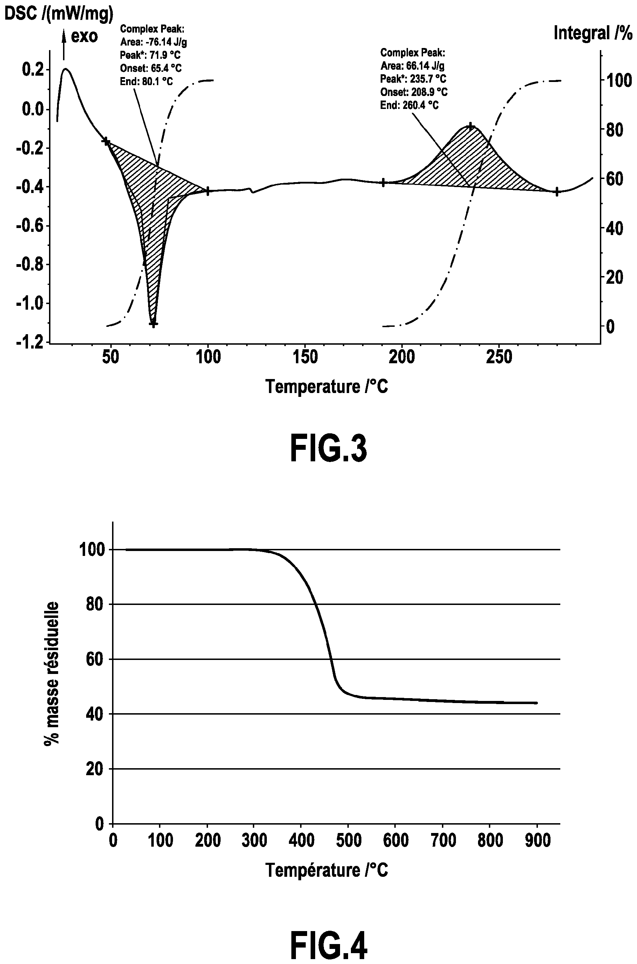

La caractérisation thermique par calorimétrie différentielle à balayage (« Differential Scanning Calorimetry » ; « DSC ») a révélé une température de fusion de 65°C ainsi qu'une réaction exothermique entre 209 et 260°C, représentant 66J/g d'enthalpie par rapport à la référence, avec une rampe de 20°C/min dans des creusets en acier scellés haute pression. Le thermogramme DSC obtenu est fourni à la figure 3. L'analyse thermogravimétrique d'un échantillon réticulé a montré un taux de coke de 46% sous atmosphère d'azote à 900°C ainsi qu'une température de dégradation de 10% de la masse totale de 404°C. Le graphe d'analyse thermogravimétrique obtenu est fourni à la figure 4 et montre de bonnes propriétés de stabilité thermique et de charbonnement.

### Exemple 3 : étude d'un mélange d'un monomère polybenzoxazine synthétisé à partir d'une polyamine avec un monomère polybenzoxazine additionnel synthétisé à partir d'un polyaldéhyde

On a synthétisé un monomère polybenzoxazine additionnel à partir d'un polyaldéhyde de la manière suivante.

On fait réagir la furfurylamine avec le salicylaldéhyde dans des proportions stoechiométriques dans le méthanol au reflux pendant 2h, dans le but de former l'imine correspondante. L'imine est réduite en amine avec 1 équivalent de NaBH₄ ajouté à 0°C dans une solution de l'imine dans le MeOH, puis on réalise un chauffage à reflux pendant 2 heures. Le furfurylaminométhylphénol ainsi synthétisé est dissous dans le toluène avec 0,5 équivalent de téréphthalaldéhyde, puis porté au reflux dans un appareil Dean Stark, afin de retirer l'eau générée pendant la réaction de condensation. La réaction est arrêtée lorsque la conversion des aldéhydes a atteint son maximum, suivi par RMN du proton. Après évaporation du solvant sous pression réduite, la bisbenzoxazine isolée est un solide blanc cassé. Le produit a été caractérisé par RMN et spectroscopie infrarouge et la structure a été confirmée.

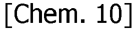

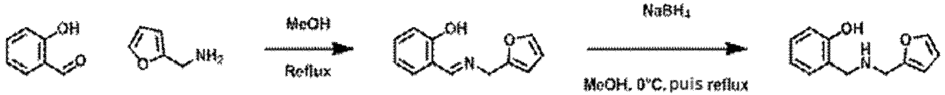

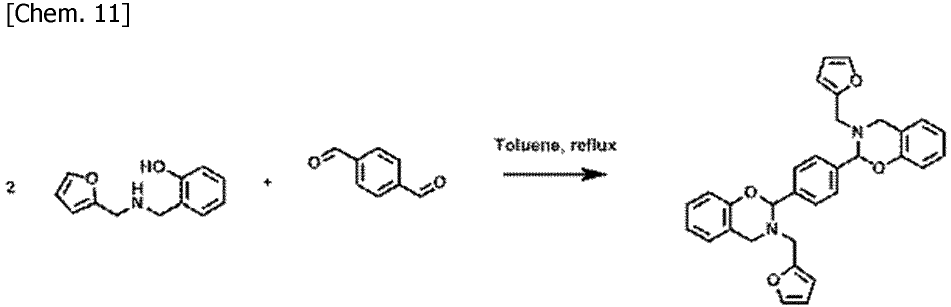

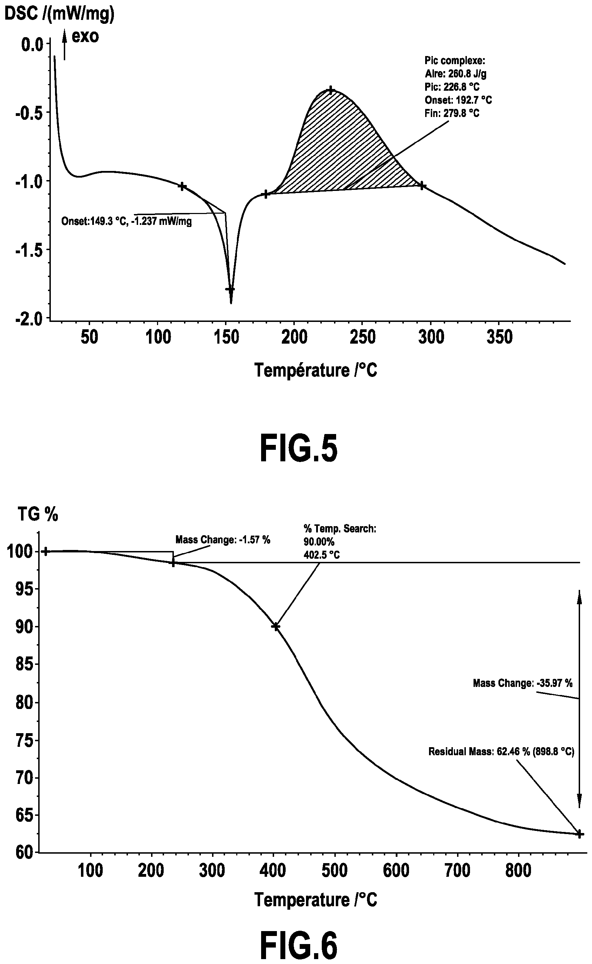

La caractérisation thermique par calorimétrie différentielle à balayage (« Differential Scanning Calorimetry » ; « DSC ») a révélé une température de fusion de 150°C ainsi qu'une réaction exothermique entre 190 et 280°C, représentant 261J/g d'enthalpie par rapport à la référence, avec une rampe de 20°C/min dans des creusets en acier scellés haute pression. Le thermogramme DSC obtenu est fourni à la figure 5.

Un échantillon de bisbenzoxazine a été réticulé à 180°C pendant 4 heures et n'a pas montré de signal résiduel en DSC. L'analyse thermogravimétrique a montré un taux de coke de 62% sous atmosphère d'azote, après 1h à 900°C ainsi qu'une température de dégradation de 10% de la masse totale de 403°C (rampe de chauffe: 20°C/min). Le graphe d'analyse thermogravimétrique obtenu est fourni à la figure 6. Le produit réticulé obtenu était insoluble dans le dichlorométhane (le taux d'insoluble après 24h à température ambiante dans le dichlorométhane, puis séchage sous vide à 60°C pendant 24h a été mesuré à hauteur de 100 +/- 0,1%).

Le monomère polybenzoxazine additionnel synthétisé à partir du polyaldéhyde a été mélangé au monomère polybenzoxazine obtenu à l'exemple 1. Le monomère polybenzoxazine additionnel synthétisé à partir du polyaldéhyde présente avantageusement, une fois réticulé, un taux de coke plus élevée par rapport à la polybenzoxazine de l'exemple 1 réticulée.

On a réalisé un mélange comprenant le monomère polybenzoxazine additionnel obtenu à partir du polyaldéhyde à raison de 25% en masse et le monomère polybenzoxazine synthétisé à partir de la polyamine à raison de 75% en masse. Le mélange a été chauffé à 80°C à l'aide d'un bain marie, puis le mélange a été homogénéisé à la spatule et ensuite réticulé par traitement thermique à 200°C.

On a réalisé une analyse thermogravimétrique de ce mélange réticulé sous azote jusqu'à 900°C. Les résultats sont fournis au tableau 1 ci-dessous. On peut remarquer que le mélange de polybenzoxazines testé possède un taux de coke intermédiaire entre les produits réticulés à partir de polybenzoxazines pures. L'adjonction du monomère polybenzoxazine additionnel synthétisé à partir du polyaldéhyde a permis d'augmenter le taux de coke. Dans le tableau 1 ci-dessous, le monomère polybenzoxazine synthétisé à partir de la polyamine est noté « bzx MXDA » et le monomère polybenzoxazine additionnel synthétisé à partir du polyaldéhyde est noté « bzx TPA ». Le mélange convient aussi pour une application en tant que résine ablative pour tuyère de propulseur.

**[Table 1]**

| **Ratio bzx MXDA/ bzx TPA** | **Taux de coke à 900°C** | **T_{d} 10%** |
|---|---|---|
| 100/0 | 57% | 448°C |
| 75 / 25 | 59% | 411°C |
| 0 / 100 | 62% | 405°C |

L'expression « compris entre ... et ... » doit se comprendre comme incluant les bornes.

## Revendications

1. Procédé de fabrication d'un monomère polybenzoxazine comprenant la condensation d'une polyamine de formule A avec un aldéhyde de formule B afin d'obtenir le monomère polybenzoxazine de formule C, les formules chimiques étant fournies ci-dessous : 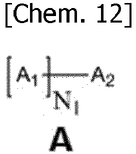 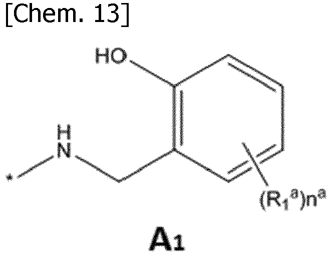 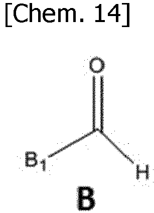 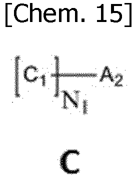 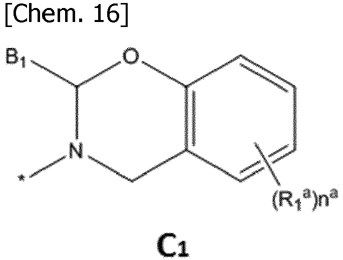 dans ces formules :
N₁ est un entier supérieur ou égal à 2,
A₂ étant un groupement xylylène substitué ou non substitué, ou étant choisi parmi les chaînes hydrocarbonées linéaires ou ramifiées, saturées, substituées ou
non substituées, non interrompues,
dans la formule A, les groupements A₁ sont identiques ou différents et présentent chacun la formule A₁ ci-dessus avec :
R₁^{a} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{a} est un entier compris entre 0 et 2,
*- désigne la liaison à A₂,
B₁ est choisi parmi : les groupes carbocycliques aromatiques ou hétérocycliques aromatiques, monocycliques ou polycycliques, substitués ou non substitués, et, lorsqu'ils sont substitués, chacun de R₁^{a}, B₁ et A₂ étant substitué par l'un au moins des groupements suivants : hydroxyméthyle, méthyle, acide carboxylique.

2. Procédé selon la revendication 1, dans lequel n^{a} est égal à 0.

3. Procédé selon la revendication 1 ou 2, dans lequel B₁ est un cycle benzénique substitué ou non substitué.

4. Procédé selon l'une quelconque des revendications 1 à 3, dans lequel N₁ est un entier égal à 2.

5. Procédé de fabrication d'un produit réticulé comprenant :
- la fabrication d'un monomère polybenzoxazine de formule C par mise en oeuvre du procédé selon l'une quelconque des revendications 1 à 4, et
- la réticulation du monomère polybenzoxazine de formule C .

6. Procédé selon la revendication 5, dans lequel il y a réticulation d'un mélange comprenant le monomère polybenzoxazine de formule C et un monomère monobenzoxazine additionnel de formule D, la formule D étant fournie ci-dessous : 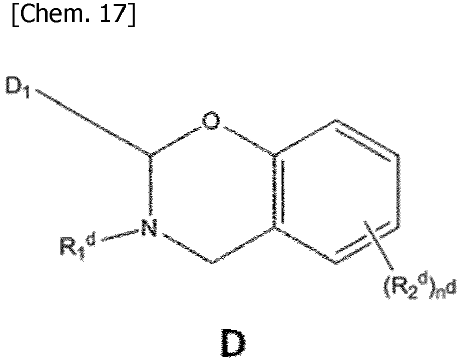 formule dans laquelle :
R₁^{d} est choisi parmi : les groupes furfuryles, substitués ou non substitués, les groupes carbocycliques ou hétérocycliques saturés, insaturés ou aromatiques, monocycliques ou polycycliques, substitués ou non substitués, les groupes aralkyles substitués ou non substitués, les chaînes hydrocarbonées linéaires ou ramifiées, saturées ou insaturées, substituées ou non substituées, interrompues ou non par un ou plusieurs hétéroatomes ou par un ou plusieurs groupes carbocycliques ou hétérocycliques saturés, insaturés ou aromatiques, monocycliques ou polycycliques, substitués ou non substitués,
R₂^{d} est choisi parmi : les groupes alcoxy, les groupes carboxyles, les atomes d'halogène, les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{d} est un entier compris entre 0 et 2,
D₁ est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes,
et, lorsqu'ils sont substitués, chacun de R₁^{d}, R₂^{d} et D₁ étant substitué par l'un au moins des groupements suivants : hydroxyméthyle, méthyle, acide carboxylique.

7. Procédé de fabrication selon la revendication 5, dans lequel il y a réticulation d'un mélange comprenant le monomère polybenzoxazine de formule C et un monomère polybenzoxazine additionnel de formule E, la formule E étant fournie ci-dessous : 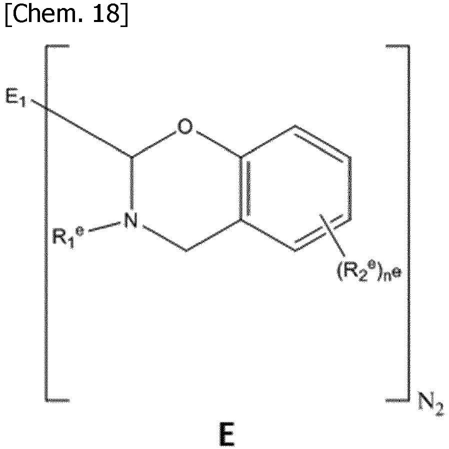
R₁^{e} est choisi parmi : les groupes furfuryles, substitués ou non substitués, les groupes carbocycliques ou hétérocycliques saturés, insaturés ou aromatiques, monocycliques ou polycycliques, substitués ou non substitués, les groupes aralkyles substitués ou non substitués, les chaînes hydrocarbonées linéaires ou ramifiées, saturées ou insaturées, substituées ou non substituées, interrompues ou non par un ou plusieurs hétéroatomes,
R₂^{e} est choisi parmi : les chaînes hydrocarbonées linéaires ou ramifiées comprenant entre 1 et 6 atomes de carbone, saturées ou insaturées, substituées ou non substituées, interrompues ou non par un ou plusieurs hétéroatomes, les groupes carbocycliques ou hétérocycliques saturés, insaturés ou aromatiques, substitués ou non substitués,
n^{e} est un entier compris entre 0 et 2,
E₁ est choisi parmi : les groupes carbocycliques ou hétérocycliques saturés, insaturés ou aromatiques, monocycliques ou polycycliques, substitués ou non substitués, les chaînes hydrocarbonées linéaires ou ramifiées, saturées ou insaturées, substituées ou non substituées, interrompues ou non par un ou plusieurs hétéroatomes, et
N₂ est un entier supérieur ou égal à 2,
et, lorsqu'ils sont substitués, chacun de E₁, R₁^{e}, R₂^{e} étant substitué par l'un au moins des groupements suivants : hydroxyméthyle, méthyle, acide carboxylique.

8. Procédé de fabrication d'une tuyère de propulseur comprenant la fabrication du produit réticulé par mise en oeuvre du procédé selon l'une quelconque des revendications 5 à 7, et la fabrication de la tuyère en mettant en oeuvre ledit produit réticulé.

9. Procédé de fabrication d'un corps de rentrée atmosphérique comprenant la fabrication du produit réticulé par mise en oeuvre du procédé selon l'une quelconque des revendications 5 à 7, et la fabrication du corps de rentrée atmosphérique en mettant en oeuvre ledit produit réticulé.

## Patentansprüche

1. Verfahren zur Herstellung eines Polybenzoxazin-Monomers, umfassend die Kondensierung eines Polyamins der Formel A mit einem Aldehyd der Formel B, um das Polybenzoxazin-Monomer mit der Formel C zu erhalten, wobei die chemischen Formeln nachstehend angegeben sind: 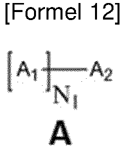 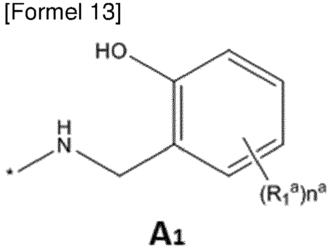 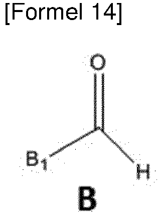 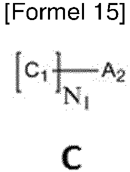 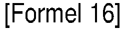 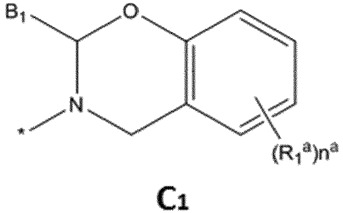 wobei in diesen Formeln:
N₁ eine ganze Zahl größer oder gleich 2 ist,
A₂ ein substituierter oder unsubstituierter Xylylenrest ist, oder aus den linearen oder verzweigten, gesättigten, substituierten oder unsubstituierten, nicht unterbrochenen Kohlenwasserstoffketten ausgewählt ist,
in der Formel A die Reste A₁ identisch oder unterschiedlich sind und jeweils die nachstehende Formel A₁ aufweisen, wobei:
R₁^{a} ausgewählt ist aus: den Alkoxygruppen, den Carboxylgruppen, den Halogenatomen, den linearen oder verzweigten, gesättigten oder ungesättigten, substituierten oder unsubstituierten, durch ein oder mehrere Heteroatome unterbrochenen oder nicht unterbrochenen Kohlenwasserstoffketten umfassend zwischen 1 und 6 Kohlenstoffatomen, den gesättigten, ungesättigten oder aromatischen, substituierten oder unsubstituierten carbocyclischen oder heterocyclischen Gruppen,
n^{a} eine ganze Zahl zwischen 0 und 2 ist,
*- die Bindung mit A₂ kennzeichnet,
B₁ ausgewählt ist aus: den aromatischen carbocyclischen oder den aromatischen heterocyclischen, monocyclischen oder polycyclischen, substituierten oder unsubstituierten Gruppen,
und, wenn sie substituiert sind, jedes von R₁^{a}, B₁ und A₂ mit mindestens einem der folgenden Reste substituiert ist: Hydroxymethyl, Methyl, Carbonsäure.

2. Verfahren nach Anspruch 1, wobei n^{a} gleich 0 ist.

3. Verfahren nach Anspruch 1 oder 2, wobei B₁ ein substituierter oder unsubstituierter Benzolring ist.

4. Verfahren nach einem der Ansprüche 1 bis 3, wobei N₁ eine ganze Zahl gleich 2 ist.

5. Verfahren zur Herstellung eines vernetzten Produkts, umfassend:
- die Herstellung eines Polybenzoxazin-Monomers der Formel C durch Umsetzung des Verfahrens nach einem der Ansprüche 1 bis 4, und
- die Vernetzung des Polybenzoxazin-Monomers der Formel C.

6. Verfahren nach Anspruch 5, bei dem es zur Vernetzung einer Mischung kommt, die das Polybenzoxazin-Monomer der Formel C und ein zusätzliches Monobenzoxazin-Monomer der Formel D umfasst, wobei die Formel D nachstehend angegeben ist: 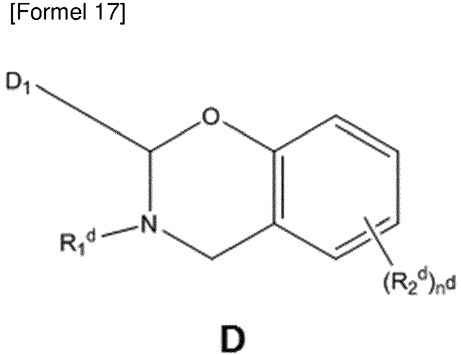 wobei in der Formel:
R₁^{d} ausgewählt ist aus: den substituierten oder unsubstituierten Furfurylgruppen, den gesättigten, ungesättigten oder aromatischen, monocyclischen oder polycyclischen, substituierten oder unsubstituierten carbocyclischen oder heterocyclischen Gruppen, den substituierten oder unsubstituierten Aralkylgruppen, den linearen oder verzweigten, gesättigten oder ungesättigten, substituierten oder unsubstituierten, durch ein oder mehrere Heteroatome oder durch eine oder mehrere gesättigte, ungesättigte oder aromatische, monocyclische oder polycyclische, substituierte oder unsubstituierte carbocyclische oder heterocyclische Gruppen unterbrochenen oder nicht unterbrochenen Kohlenwasserstoffketten,
R₂^{d} ausgewählt ist aus: den Alkoxygruppen, den Carboxylgruppen, den Halogenatomen, den linearen oder verzweigten, gesättigten oder ungesättigten, substituierten oder unsubstituierten, durch ein oder mehrere Heteroatome unterbrochenen oder nicht unterbrochenen Kohlenwasserstoffketten umfassend zwischen 1 und 6 Kohlenstoffatomen, den gesättigten, ungesättigten oder aromatischen, substituierten oder unsubstituierten carbocyclischen oder heterocyclischen Gruppen,
n^{d} eine ganze Zahl zwischen 0 und 2 ist,
D₁ ausgewählt ist aus: den gesättigten, ungesättigten oder aromatischen, monocyclischen oder polycyclischen, substituierten oder unsubstituierten carbocyclischen oder heterocyclischen Gruppen, den linearen oder verzweigten, gesättigten oder ungesättigten, substituierten oder unsubstituierten, durch ein oder mehrere Heteroatome unterbrochenen oder nicht unterbrochenen Kohlenwasserstoffketten,
und, wenn sie substituiert sind, jedes von R₁^{d}, R₂^{d} und D₁ mit mindestens einem der folgenden Reste substituiert ist: Hydroxymethyl, Methyl, Carbonsäure.

7. Herstellungsverfahren nach Anspruch 5, bei dem es zur Vernetzung einer Mischung kommt, die das Polybenzoxazin-Monomer der Formel C und ein zusätzliches Polybenzoxazin-Monomer der Formel E umfasst, wobei die Formel E nachstehend angegeben ist: 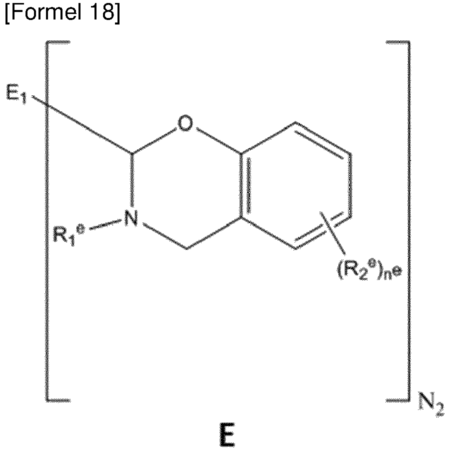
R₁^{e} ausgewählt ist aus: den substituierten oder unsubstituierten Furfurylgruppen, den gesättigten, ungesättigten oder aromatischen, monocyclischen oder polycyclischen, substituierten oder unsubstituierten carbocyclischen oder heterocyclischen Gruppen, den substituierten oder unsubstituierten Aralkylgruppen, den linearen oder verzweigten, gesättigten oder ungesättigten, substituierten oder unsubstituierten, durch ein oder mehrere Heteroatome unterbrochenen oder nicht unterbrochenen Kohlenwasserstoffketten,
R₂^{e} ausgewählt ist aus: den linearen oder verzweigten, gesättigten oder ungesättigten, substituierten oder unsubstituierten, durch ein oder mehrere Heteroatome unterbrochenen oder nicht unterbrochenen Kohlenwasserstoffketten umfassend zwischen 1 und 6 Kohlenstoffatomen, den gesättigten, ungesättigten oder aromatischen, substituierten oder unsubstituierten, carbocyclischen oder heterocyclischen Gruppen,
n^{e} eine ganze Zahl zwischen 0 und 2 ist,
E₁ ausgewählt ist aus: den gesättigten, ungesättigten oder aromatischen, monocyclischen oder polycyclischen, substituierten oder unsubstituierten carbocyclischen oder heterocyclischen Gruppen, den linearen oder verzweigten, gesättigten oder ungesättigten, substituierten oder unsubstituierten, durch ein oder mehrere Heteroatome unterbrochenen oder nicht unterbrochenen Kohlenwasserstoffketten, und
N₂ eine ganze Zahl größer oder gleich 2 ist,
und, wenn sie substituiert sind, jedes von E₁, R₁^{e} und R₂^{e} mit mindestens einem der folgenden Reste substituiert ist: Hydroxymethyl, Methyl, Carbonsäure.

8. Verfahren zur Herstellung einer Schubdüse, umfassend die Herstellung des vernetzten Produkts durch Umsetzung des Verfahrens nach einem der Ansprüche 5 bis 7 und die Herstellung der Düse durch Verwenden des vernetzten Produkts.

9. Verfahren zur Herstellung eines Atmosphären-Wiedereintrittskörpers, umfassend die Herstellung des vernetzten Produkts durch Umsetzung des Verfahrens nach einem der Ansprüche 5 bis 7 und die Herstellung des Atmosphären-Wiedereintrittskörpers durch Verwenden des vernetzten Produkts.

## Claims

1. A process for manufacturing a polybenzoxazine monomer comprising condensing a polyamine of formula A with an aldehyde of formula B in order to obtain the polybenzoxazine monomer of formula C, the chemical formulas being provided below: 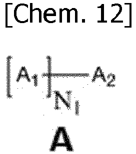 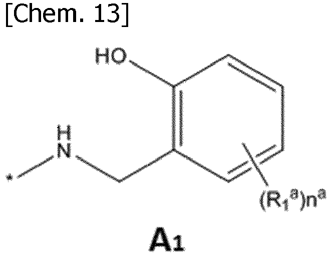 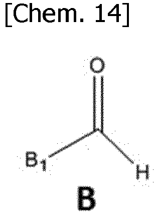 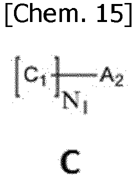 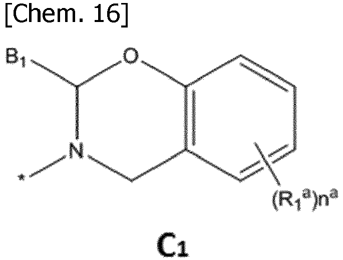 in these formulas:
N₁ is an integer greater than or equal to 2;
A₂ is a substituted or unsubstituted xylylene group, or being selected from linear or branched, saturated, substituted or unsubstituted hydrocarbon chains that are uninterrupted;
in formula A, the groups A₁ are identical or different and each have the above formula A₁ with:
R₁^{a} is selected from: alkoxy groups; carboxyl groups; halogen atoms; saturated or unsaturated, substituted or unsubstituted, linear or branched hydrocarbon chains comprising between 1 and 6 carbon atoms, optionally interrupted by one or more heteroatoms; saturated, unsaturated or aromatic, substituted or unsubstituted carbocyclic or heterocyclic groups;
n^{a} is an integer comprised between 0 and 2;
*- designates the bond to A₂;
B₁ is selected from: aromatic, monocyclic or polycyclic, substituted or unsubstituted carbocyclic or heterocyclic groups;
and, when they are substituted, each of R₁^{a}, B₁ and A₂ being substituted with at least one of the following groups: hydroxymethyl, methyl, carboxylic acid.

2. The process as claimed in claim 1, wherein n^{a} is equal to 0.

3. The process as claimed in claim 1 or 2, wherein B₁ is a substituted or unsubstituted benzene ring.

4. The process as claimed in any one of claims 1 to 3, wherein N₁ is an integer equal to 2.

5. A process for manufacturing a crosslinked product comprising:
- manufacturing a polybenzoxazine monomer of formula C by carrying out the process as claimed in any one of claims 1 to 4, and
- crosslinking the polybenzoxazine monomer of formula C.

6. The process as claimed in claim 5, wherein there is crosslinking of a mixture comprising the polybenzoxazine monomer of formula C and an additional monobenzoxazine monomer of formula D, formula D being provided below: 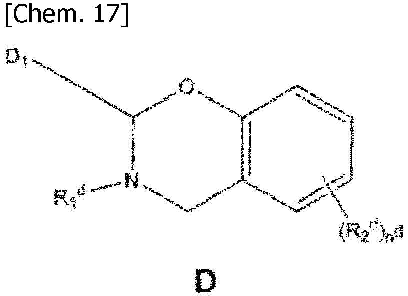 formula in which:
R₁^{d} is selected from: substituted or unsubstituted furfuryl groups; saturated, unsaturated or aromatic, monocyclic or polycyclic, substituted or unsubstituted carbocyclic or heterocyclic groups; substituted or unsubstituted aralkyl groups; saturated or unsaturated, substituted or unsubstituted, linear or branched hydrocarbon chains, optionally interrupted by one or more heteroatoms or by one or more saturated, unsaturated or aromatic, monocyclic or polycyclic, substituted or unsubstituted carbocyclic or heterocyclic groups;
R₂^{d} is selected from: alkoxy groups; carboxyl groups; halogen atoms; saturated or unsaturated, substituted or unsubstituted, linear or branched hydrocarbon chains comprising between 1 and 6 carbon atoms, optionally interrupted by one or more heteroatoms; saturated, unsaturated or aromatic, substituted or unsubstituted carbocyclic or heterocyclic groups;
n^{d} is an integer comprised between 0 and 2;
D₁ is selected from: saturated, unsaturated or aromatic, monocyclic or polycyclic, substituted or unsubstituted carbocyclic or heterocyclic groups; linear or branched, saturated or unsaturated, substituted or unsubstituted hydrocarbon chains,
optionally interrupted by one or more heteroatoms;
and, when they are substituted, each of R₁^{d}, R₂^{d} and D₁ being substituted with at least one of the following groups: hydroxymethyl, methyl, carboxylic acid.

7. The manufacturing process as claimed in claim 5, wherein there is crosslinking of a mixture comprising the polybenzoxazine monomer of formula C and an additional polybenzoxazine monomer of formula E, formula E being provided below: 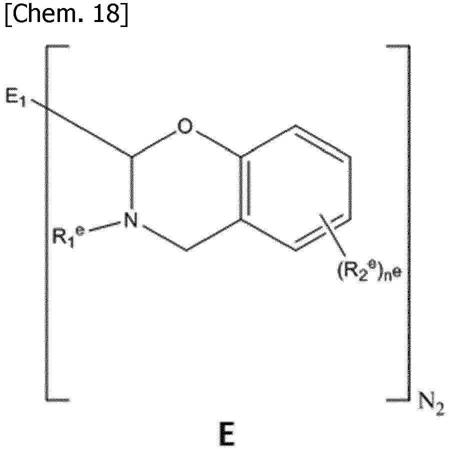
R₁^{e} is selected from: substituted or unsubstituted furfuryl groups; saturated, unsaturated or aromatic, monocyclic or polycyclic carbocyclic or heterocyclic groups, substituted or unsubstituted, substituted or unsubstituted aralkyl groups; substituted or unsubstituted, linear or branched, saturated or unsaturated, substituted or unsubstituted hydrocarbon chains, optionally interrupted by one or more heteroatoms;
R₂^{e} is selected from: saturated or unsaturated, substituted or unsubstituted, linear or branched hydrocarbon chains comprising between 1 and 6 carbon atoms, optionally interrupted by one or more heteroatoms; saturated, unsaturated or aromatic, substituted or unsubstituted carbocyclic or heterocyclic groups;
n^{e} is an integer comprised between 0 and 2;
E₁ is selected from: saturated, unsaturated or aromatic, monocyclic or polycyclic, substituted or unsubstituted carbocyclic or heterocyclic groups; linear or branched, saturated or unsaturated, substituted or unsubstituted hydrocarbon chains,
optionally interrupted by one or more heteroatoms; and
N₂ is an integer greater than or equal to 2,
and, when they are substituted, each of E₁, R₁^{e}, R₂^{e} being substituted with at least one of the following groups: hydroxymethyl, methyl, carboxylic acid.

8. A process for manufacturing a thruster nozzle comprising manufacturing the crosslinked product by carrying out the process as claimed in any one of claims 5 to 7 and manufacturing the nozzle by using said crosslinked product.

9. A process for manufacturing an atmospheric reentry body comprising manufacturing the crosslinked product by carrying out the process as claimed in any one of claims 5 to 7 and manufacturing the atmospheric reentry body by using said crosslinked product.
